# EUROPEAN PATENT APPLICATION

(11) **EP 2 253 349 A1**
(43) Date of publication of application: **24.11.2010**
(21) Application number: 09712289.9
(22) Date of filing: 19.02.2009
(51) Int. Cl.: A61M 5/28

(54) **COMBINATION SYRINGE/CONTAINER**

(30) Priority: 19.02.2008 JP 2008038006; 10.03.2008 JP 2008059590; 24.09.2008 JP 2008244182
(71) Applicant: ARTE CORPORATION, Shiyoda-ku Tokyo 101-0032 (JP)
(72) Inventor: SHIMAZAKI, Seiji, Takahagi-shi Ibaraki 318-0004 (JP); KAKIUCHI, Makoto, Takahagi-shi Ibaraki 318-0004 (JP)
(74) Representative: De Vries & Metman
(86) International application number: PCT/JP2009/052909
(87) International publication number: WO 2009/104686

(57) **Abstract**

In a combined container-syringe (1), a rubber packing (7) that covers the distal end opening of a cylinder (2) at a fitting hole (3d) of a cylindrical tip (3) in an air tight and liquid tight manner, and in which is formed a through hole (7e) that brings a communication hole (3f) formed in a lure tip (3a) and the inside of the cylinder (2) into communication, and a rubber member (8) that is inserted from the distal end side of the communication hole (3f) and blocks the through hole (7e) are provided, and the rubber packing (7) is formed with an elastic body that is corrosion resistant to the liquid medicament. With this combined container-syringe (1), in addition to being able to store it for a long period without deteriorating the liquid medicament, it is possible to observe well the liquid medicament or blood that flows to the cylindrical tip, and moreover preventing leftover liquid medicament after injection becomes possible.

## Description

### TECHNICAL FIELD

The present invention relates to a combined container-syringe that is pre-filled with a liquid medicament and stored, and when used can be removed from the packaging and used immediately.

Priority is claimed on Japanese Patent Application No. 2008-38006, filed in Japan on February 19, 2008, Japanese Patent Application No. 2008-59590, filed in Japan on March 10, 2008, and Japanese Patent Application No. 2008-244182, filed in Japan on September 24, 2008, the content of which is incorporated herein by reference.

### BACKGROUND ART

Since a combined container-syringe is pre-filled with a liquid medicament, it can be used immediately after being removed from the packaging without performing a complicated operation at a medical institution. Therefore, it has outstanding convenience and is extremely useful in lightening the workload of doctors and nurses, and for this reason has been adopted in many hospitals and clinics.

As this combined container-syringe, there is known one that is provided with a cylinder, a front stopper and a rear stopper that are fitted to the front and rear end sides in the cylinder and seal in a liquid tight manner the liquid medicament that is filled in the cylinder, a cylindrical tip provided with a bypass chamber in which the front stopper enters and a lure tip for attaching a needle of the distal end thereof, a finer grip that is attached to the rear end of the cylinder, and a plunger rod that is inserted from the rear end portion of the cylinder to the inside of the cylinder and coupled to the rear stopper (for example, refer to Patent Documents 1 and 2).

During use, when the rear stopper is pushed by the plunger rod and the front stopper is pushed forward together with the liquid medicament, the front stopper is pushed out from the cylinder and enters the bypass chamber of the cylindrical tip. Thereby, the liquid medicament that is hermetically sealed between the rear and front stoppers, by releasing the hermetic seal at the distal end side, flows out from within the cylinder to the inside of the bypass chamber, and this liquid medicament flows through the gap between the front stopper and the bypass chamber and is guided to the inner surface of the lure tip along a bypass groove that is provided in the inner wall of the bypass chamber and introduced to the needle.

Also, when unused, the liquid medicament is filled between the front and rear stoppers in the cylinder and retained. Here, the cylinder is made of glass and the front and rear stoppers are made of butyl rubber. Since these materials have been proven to be chemically stable with respect to a liquid medicament, they can be stably used as a container that stores the liquid medicament. Accordingly, it is possible to store the syringe serving as a storage container over a long period without the liquid medicament deteriorating.

In a conventional combined container-syringe, since the cylinder and the front and rear stoppers are air tightly and liquid tightly fitted together, the liquid medicament that is filled and sealed therein is not at all affected by oxygen in the air.

Also, since silicone oil is uniformly and at a minimum required amount baked at a high temperature onto the inner wall of the cylinder, even after storage for a long time, the front and rear stoppers can smoothly slide in the cylinder. Note that when such a cylinder oil is backed on, since it can be heated at a high temperature of 250°C or higher, simultaneously the cylinder is hot air sterilized and can be made pyrogen free and endotoxin free.

Above are the characteristics of a glass cylinder which cannot be accomplished in a cylinder that is made of plastic.

On the other hand, in the case of using one in which the cylindrical tip is made of glass, there are the following shortcomings.

That is, the outer diameter of the lure tip that attaches the needle has a diameter of around 4 mm, and a flow exit with a diameter of 1.0 to 1.2 mm must be provided in the axis center, but in the case of glass, it is extremely difficult to form the shape and dimensions to specification by melting and molding the distal end of the cylindrical tip, and expert technique is required.

Also, since the cylindrical tip that the lure tip that are formed by glass have an outer diameter of as thin as 4 mm, they are easily broken by a slight force or impact and are difficult to manufacture. When handling during injection as well, special attention must be paid to the handling so that the cylindrical tip does not break.

Moreover, when the needle is attached, the glass cylindrical tip, having the characteristics of glass, easily slips, and the needle sometimes comes out from the cylindrical tip with the needle base during injection. In particular, in the case of a silicone treatment done on the outer cylinder, when even a little silicone oil adheres, during attachment of the needle it becomes slippery, and the needle may easily come out.

Accordingly, in a conventional syringe serving as a container, use of a glass cylindrical tip that has the above shortcomings has been avoided, and a plastic cylindrical tip has been adopted.

When the cylindrical tip is fabricated with plastic, the flexibility of design is high, so that even a lure lock that has a complicated and challenging shape can be fabricated provided a metal mold can be made. Moreover, depending on the selection of materials, it is possible to fabricate a cylindrical tip that is strong and provided with shock resistance.

Also, since there is some resiliency in the material, even when the needle is attached, there is a stability in terms of the material in joining the needle with the needle base, and the needle does not easily come out during an injection. At this time, for a cylindrical tip that is provided with the lure lock portion, it is possible to enhance the feeling of unity, and so there is a high degree of security.

In the above manner, in a conventional combined container-syringe, by combining the material benefits of glass and plastic depending on the purpose of use, a safe and easy to use combined container-syringe is realized.
Patent Document 1: Japanese Patent (Granted) Publication No. S62-58745
Patent Document 2: Japanese Patent Application, First Publication No. H08-141081

An injection is a medical act of injecting a liquid medicament into a necessary site in a body, and depending on the type, efficacy and purpose of use of the liquid medicament, the puncture site is stipulated, and for example is divided into types such as an intravenous injection, an artery injection, a hypodermic injection, and an endermic injection.

Accordingly, it is clear that a mistake in the puncture site will lead to a serious medical accident, and in order to prevent such a danger beforehand, after making the puncture with the needle at the medical treatment location, the plunger rod is once pulled toward oneself without, immediately injecting the liquid medicament so as to create negative pressure inside of the syringe. Thereby, in the event of the cutting edge of the needle being in a blood vessel, a small amount of blood will flow into the syringe (flashback), and so it is possible to confirm that the cutting edge is in a blood vessel. This procedure is generally called nurse aspiration.

In order to perform this procedure, when negative pressure is applied to the syringe, it must be possible to immediately confirm visually the existence of a backflow of blood into the syringe and the color state thereof, and for this reason it must be possible to be able to readily confirm the existence of an inflow and the color state at the cylindrical tip of the syringe.

Also, this is not limited to nurse aspiration, and in the case of performing a mixed injection that involves drawing separate medical products into the syringe, mixing two or more medical products and injecting them, it is desired to be able to confirm the inflow of the liquid medicament at the cylindrical tip of the syringe.

However, in the conventional combined container-syringe as outlined above, due to the existence of the front and rear stoppers, there is little spatial room to be able to visually confirm the existence and color state of the inflow of blood and liquid medicament during nurse aspiration and mixed injection, and so because of this situation an improvement is required to readily perform the aforementioned confirmation.

Even if a liquid medicament in the cylinder is introduced to the needle side by completely pushing in the plunger rod, since the bypass channel in the bypass chamber allows leftover liquid medicament, there is the problem of wasted liquid medicament occurring that is not administered to the patent.

The present invention has been achieved in view of the above circumstances, and has as its object to provide a combined container-syringe that is as safe and easy to use as a conventional combined container-syringe and capable of being stored for a long time without degrading a liquid medicament, and also allows good observation of the liquid medicament or blood that flows into the cylindrical tip, and moreover is capable of preventing leftover liquid medicament after injection.

### DISCLOSURE OF THE INVENTION

In order to solve the above issues, the present invention provides the following means.

That is, a combined container-syringe according to the present invention relates to a combined container-syringe provided with a cylinder made of glass that has a cylindrical shape; a stopper that is fitted into a rear end side of the cylinder in a liquid tight manner; a cylindrical tip that is formed with a transparent synthetic resin material, fitted from an outer side on a distal end outer periphery of the cylinder via a fitting hole on a base end side, and provided with a lure tip to which a needle is attached at the distal end side and in which a communication hole that communicates with the inside of the cylinder is formed; a finger grip that is provided at the rear end of the cylinder; and a plunger rod that is inserted in the cylinder from the rear end side of the cylinder and coupled to the stopper. In the combined container-syringe of the present invention, a rubber packing that covers the distal end opening of the cylinder at the fitting hole in an air tight and liquid tight manner and in which a through hole that brings the communication hole and the inside of the cylinder into communication is formed, and a sealing member that is inserted from the distal end side of the communication hole and blocks the through hole are provided, and the stopper and the rubber packing are formed with elastic bodies that are corrosion resistant to the liquid medicament. The liquid medicament is filled between the stopper and the rubber packing in the cylinder.

Thereby, the liquid medicament that is in the combined container-syringe when unused, besides making slight contact with the sealing member, is enclosed by the glass cylinder, and the stopper and the rubber packing that are corrosion resistant to the liquid medicament.

Therefore, the liquid medicament comes to be surrounded by the cylinder, the stopper and the rubber packing that are all chemically stable with respect to the liquid medicament and in which elution and the like does not occur. For that reason, there is no deterioration in the quality of the liquid medicament even if the combined container-syringe is stored for a long period.

Also, as for the liquid medicament in the combined container-syringe in this way, since the distal end side is held in a liquid tight manner by the rubber packing and the sealing member, and it is possible to introduce the liquid medicament to the needle via the communication route of the lure tip by only removing the sealing member during use, performing holding and introduction of the liquid medicament with a simple constitution becomes possible.

Accordingly, there is no need to provide special constituent elements such as a bypass chamber and a front stopper in conventional syringes also serving as a container. For that reason, the visual recognition in the cylindrical tip is not hindered, and leftover liquid medicament after injection is not allowed.

Also, in the combined container-syringe according to the present invention, a peripheral edge of the through hole in the rubber packing may extend to the inside of the communication hole, and cover the inner peripheral surface, of the communication hole.

The liquid medicament in the combined container-syringe during use passes the communication hole of the cylindrical tip to be introduced to the needle. However, at this time, the inner periphery surface of the communication hole is covered by the rubber packing that has corrosion resistance, and the liquid medicament passes through the inside of the rubber packing. Accordingly, introducing the liquid medicament to the needle while maintaining the quality without degrading the liquid medicament becomes possible.

Moreover, in the combined container-syringe according to the present invention, the sealing member may be made of a synthetic resin that is corrosion resistant to the liquid medicament.

In the storage state of not being used, the liquid medicament makes slight contact with the sealing member, but since the sealing member is formed with a synthetic resin that has corrosion resistance, it is possible to suppress to a minimum the impact on the liquid medicament, and so maintaining the quality of the liquid medicament becomes possible.

Moreover, in the combined container-syringe according to the present invention, it is preferable that the front end portion of the cylinder extends toward the inner side in the radial direction of the cylinder, whereby the distal end opening portion of the cylinder contracts in diameter, and the rubber packing, by making contact with the entire region of the front end portion of the cylinder, covers the distal end opening of the cylinder in an air tight and liquid tight manner.

According to the combined container-syringe with such characteristics, it is possible to increase the contact surface area between the rubber packing and the front end portion of the cylinder. For that reason, it is possible to enhance the air tightness and the liquid tightness of the liquid medicament in the cylinder, and so reliably preventing the liquid medicament from leaking or outside air from intruding into the cylinder becomes possible.

Moreover, in the combined container-syringe according to the present invention, a constitution may be one in which a sealing member that blocks the communication hole is provided, and the sealing member and the stopper are formed with elastic bodies that are corrosion resistant to the liquid medicament, at least the contact location with the liquid medicament at the cylindrical tip is formed with a synthetic resin that is corrosion resistant to the liquid medicament, and the liquid medicament is filled between the stopper in the cylinder and the sealing member and the cylindrical tip.

According to the combined container-syringe with such characteristics, when unused, the liquid medicament is surrounded by materials that are chemically stable with respect to the liquid medicament and do not impart an influence by elution and the like. For that reason, there is no deterioration in the quality of the liquid medicament even if the combined container-syringe is stored for a long period.

Also, as for the liquid medicament in the combined container-syringe in this way, since the distal end side is held in a liquid tight manner by only the cylindrical tip and the sealing member, and it is possible to introduce the liquid medicament to the needle through the communication route of the lure tip simply by removing the sealing member, performing holding and introduction of the liquid medicament with a simple constitution becomes possible.

Accordingly, since there is no need to provide special constituent elements such as a bypass chamber and a front stopper in conventional syringes also serving as a container, the visual recognition in the cylindrical tip is not hindered, and leftover liquid medicament after injection is not allowed.

Moreover, in the combined container-syringe according to the present invention, the seating member may be one that covers the entire region of the outer peripheral surface of the cylindrical tip.

Thereby, it is possible to further increase the air tightness and liquid tightness at the cylindrical tip distal end, and so completely preventing leakage of the liquid medicament and intrusion of outside air during storage becomes possible.

Also, in the combined container-syringe according to the present invention, the stopper and the rubber packing, or the sealing member are preferably made of butyl rubber.

Since butyl rubber exhibits good stability toward a liquid medicament and has high corrosion resistance, it does not exert an adverse impact on the liquid medicament even when in contact with the liquid medicament for a long period, and so it is possible to achieve maintenance of the quality of the liquid medicament. For that reason, effectively suppressing deterioration of the quality of the liquid medicament and storing the combined container-syringe for an even longer period becomes possible.

Also, in the combined container-syringe according to the present invention, the aforementioned synthetic resin is preferably any one of polypropylene, annular polyolefin resin, and annular polyolefin copolymer.

Since these synthetic resins are chemically stable with respect to the liquid medicament and exhibit high corrosion resistance, suppressing deterioration of the liquid medicament becomes possible.

### [Effect of the Invention]

According to the combined container-syringe of the present invention, since it is possible to enclose the liquid medicament with members such as the glass cylinder, the stopper and the rubber packing or the scaling member that are all corrosion resistant to the liquid medicament, it can be stored for a long period without deteriorating the liquid medicament.

Also, since there are no members that hinder visual recognition in the cylindrical tip, observation of the inflow of liquid medicament or blood that flows into the cylindrical tip is made easy, and so preventing leftover liquid medicament after use becomes possible.

### BRIEF DESCRIPTION OF THE DRAWINGS

FIG. 1 is a side cross-sectional view of the combined container-syringe according to one embodiment of the present invention.
FIG. 2 is an essential portion enlarged view of FIG. 1.
FIG. 3 is a drawing that shows the essential portions of the first modification.
FIG. 4 is a drawing that shows the essential portions of the second modification.
FIG. 5 is a drawing that shows the essential portions of the third modification.
FIG. 6 is a drawing that shows the essential portions of the fourth modification.
FIG. 7 is a drawing that shows the essential portions of the combined container-syringe according to the second embodiment of the present invention.
FIG. 8 is a drawing that shows the essential portions of a modification of the second embodiment.

### [Description of Reference Numerals]

1, 30 combined container-syringe, 2 cylinder, 2c front end portion, 3 cylindrical tip, 3a lure tip, 3b base end portion, 3c lure lock portion, 3d fitting hole, 3f, 31f communication hole, 4 finger grip, 5 stopper, 6 plunger rod, 7 rubber packing, 7e through hole, 8 rubber cap (sealing member), 8c sealing portion, 10 synthetic resin cap (sealing member), 10c sealing portion, 11 sealing rubber (sealing member), 12 fixing cap, 31 integrated cylindrical tip

### BEST MODE FOR CARRYING OUT THE INVENTION

Hereinbelow, the combined container-syringe according to the embodiments of the present invention shall be described with reference to the drawings.

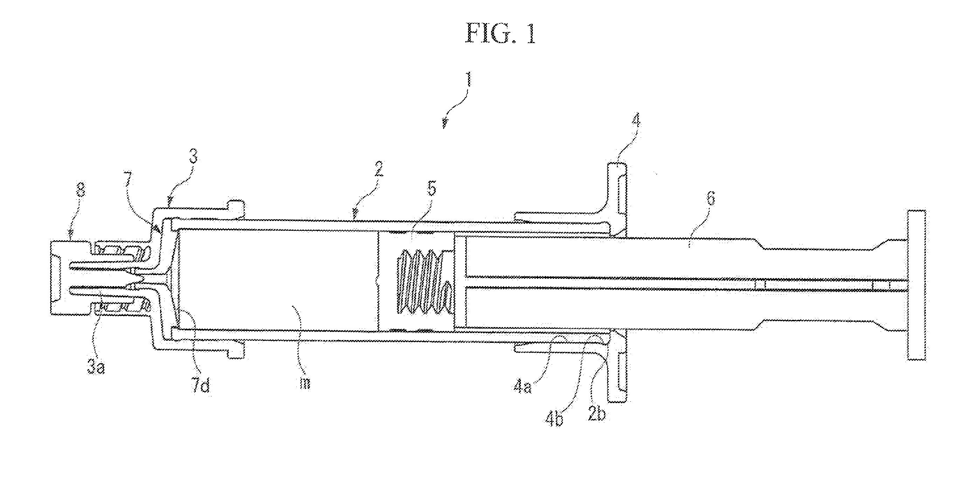

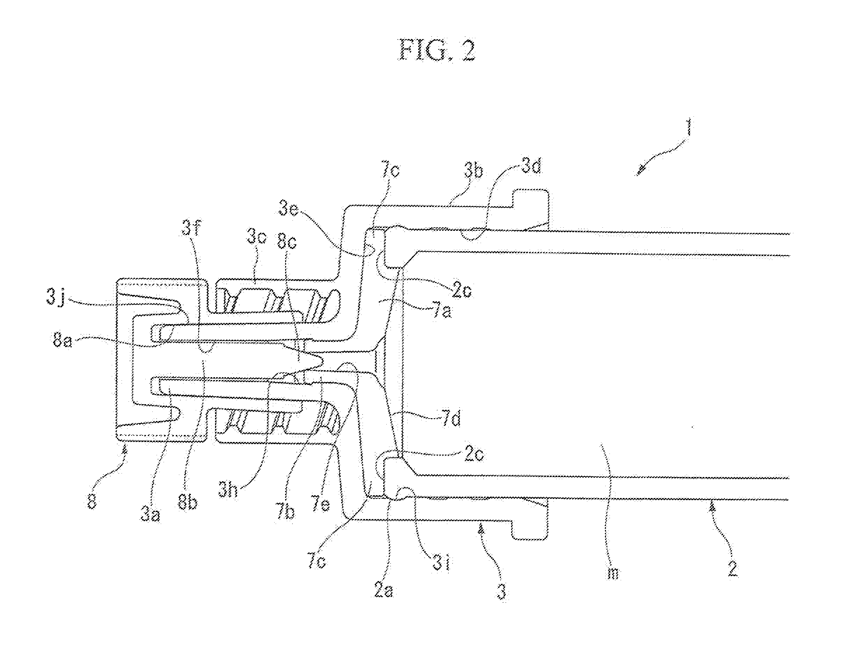

FIG. 1 is a side cross-sectional view of the combined container-syringe according to a first embodiment of the present invention, and FIG. 2 is a partial enlarged view of the vicinity of the distal end in FIG. 1.

As shown in FIG. 1, a combined container-syringe 1 is provided with a cylinder 2, a cylindrical tip 3 that is fitted from the outer side onto the outer periphery of the front end (the left side in FIG. 1) of the cylinder 2, a finger grip 4 made of a synthetic resin that is attached to the outer periphery of the rear end (right side in FIG. 1) of the cylinder 2, a stopper 5 that seals the liquid medicament m that is filled in the cylinder 2 from the rear end side, and a plunger shaft 6 that is inserted in the cylinder 2 from the rear end side with the distal end portion coupled to the stopper 5, and that makes the stopper 5 move back and forth in the axial direction of the cylinder 2.

Moreover, the combined container-syringe 1 is provided with a rubber packing 7 that covers the front end of the cylinder 2 in the cylindrical tip 3 and a rubber cap 8 that is inserted in the lure tip 3a that is provided at the distal end of the cylindrical tip 3.

The cylinder 2 has a cylindrical shape that is made of transparent glass, and the liquid medicament in is filled in the interior thereof with the stopper 5 fitted so as to enclose the liquid medicament m from the rear end side.

The stopper 5 has a columnar shape that has an outer diameter that is slightly larger than the inner diameter of the cylinder 2, and is formed from an elastic body that is corrosion resistant to the liquid medicament, for example, rubber for medical applications and pharmaceutical applications (butyl rubber in the present embodiment). This stopper 5 is fitted in the cylinder 2 so as to be positioned slightly to the rear end side from the center portion in the axial direction of the cylinder 2.

As for the cylindrical tip 3, as shown in detail in FIG. 2, the outer shape has a two-stage columnar shape that is constituted from a transparent, synthetic resin that is provided with suitable rigidity, and is provided with a base end portion 3b with a cylindrical shape, a lure tip 3 a with a comparatively small diameter that is joined to the front side of the base end portion 3b, and a lure lock portion 3c that is spaced to the outer side of the lure tip 3a in the radial direction and joined to the front side of the base end portion 3b.

A bottomed fitting hole 3d is formed on the inner side of the base end portion 3b, and by fitting the fitting hole 3d on the outer periphery of the distal end of the cylinder 2 from the outer side, the cylindrical tip 3 is attached in an air tight and liquid tight manner on the front end portion of the cylinder.

Also, the outer peripheral surface of the lure tip 3a is made into a tapered outer peripheral surface 3j that contracts in diameter while heading to the distal end side, and a communication hole 3f that is continuous with a bottom portion 3e of the 3d is formed penetrating in the front/rear direction through the inside of the lure tip 3a along the axis line.

Furthermore, a lock screw 3g for engaging the rubber cap 8 mentioned below or the needle base of the needle not illustrated is formed in the above-mentioned lure lock portion 3c.

Note that a ring-shaped projection 2a is provided at the distal end outer peripheral portion of the abovementioned cylinder 2, and by fitting the projection 2a in a ring-shaped groove 3i that is formed at the front end side of the fitting hole 3d of the cylindrical tip 3, the cylindrical tip 3 is attached to the distal end of the cylinder 2 in an air tight and liquid tight manner. Moreover, by the front end portion 2c of the cylinder 2 extending toward the inner side in the radial direction of the cylinder 2, the opening portion at the distal end of the cylinder 2 contracts in diameter.

Also, at the rear end outer peripheral portion of the cylinder 2, as shown in FIG. 1, a projection 2b with the same ring shape as the projection 2a is provided, and by fitting the projection 2b in a ring-shaped groove 4b that is formed in a cylindrical hole portion 4a of the finger grip 4, the finger grip 4 is firmly attached to the cylinder 2.

Note that in addition to the constitution of the finger grip 4 being separately attached to the cylinder 2, it may be one that is integrally formed with a cylinder 22.

Then, in the present embodiment, the rubber packing 7 that covers the distal end side opening of the cylinder 2 is provided within the fitting hole 3d of the cylindrical tip 3 as mentioned above.

The rubber packing 7 consists of rubber for medical applications and pharmaceutical applications (butyl rubber in the present embodiment), and is constituted from a disk portion 7a that is formed with an outer diameter that is approximately the same as the abovementioned cylinder, and a protruding portion 7b that extends so as to protrude from the center of the disk portion 7a toward the inside of the communication hole 3f of the aforementioned lure tip 3.

The surface of the abovementioned disk portion 7a that faces the rear end side of the cylinder 2 gently slopes from an outer edge portion 7c thereof toward the center to be concaved to the distal end side, and thus is made into an abutment surface 7d that the stopper 5 abuts when the injection is completed. Also, a through hole 7e that penetrates the center of the disk portion 7a and the inside of the protruding portion 7b along the axis line is formed in this rubber packing 7, and the through hole 7e is opened to the center of the aforementioned abutment surface 7d.

The rubber packing 7 of this kind of constitution, by fitting the cylindrical tip 3 on the cylinder 2 from the outer side, is fixed by the outer edge portion 7c of the disk portion 7a being sandwiched by the front end portion 2c of the cylinder 2c and the bottom portion 3e of the fitting hole 3d in the cylindrical tip 3, and covers the distal end opening portion of the cylinder 2.

By thus isolating the inner portion of the cylinder 2 and the cylindrical tip 3 with the rubber packing 7 in an air tight and liquid tight manner, the liquid medicament m that is filled in the cylinder 2 is prevented from making direct contact with the fitting hole 3d and the bottom portion 3e of the cylindrical tip 3.

Note that the outer edge portion 7c serves as assistance in maintaining the fitting of the cylindrical tip 3 and the cylinder 2 air tight and liquid tight, and plays a role of doubly preventing the internal liquid medicament m from leaking or outside air from intruding.

In particular, since the rubber packing 7 covers the distal end opening of the cylinder 2 in an air tight and liquid tight manner by making contact with the entire region of the front end portion 2c that extends to the inner side in the radial direction of the cylinder 2, it is possible to enlarge the contact surface area between the rubber packing 7 and the front end portion 2c of the cylinder 2. Thereby, by improving the air tightness and liquid tightness of the liquid medicament in in the cylinder 2, it is possible to securely prevent the liquid medicament m from leaking or outside air from entering into the cylinder 2.

Also, when the rubber packing 7 is fixed in the cylindrical tip 3, the protruding portion 7b thereof enters into the inside of the communication hole 3f of the lure tip 3a, and a projection 7f that is provided on the distal end outer periphery of the protruding portion 7b engages with an engagement portion 3h that is formed in the communication hole 3f of the lure tip 3a. Thereby, the protruding portion 7b of the rubber packing 7 is fixed to the communication hole 3f so as not to shift by for example the insertion of an insertion portion 8b of the rubber cap 8 described below.

In addition, in the present embodiment, the above-mentioned protruding portion 7b is formed with a length that is for example approximately one-fourth the total length of the communication hole 3f in the lure tip 3a.

The rubber cap 8 consists of rubber for medical applications and pharmaceutical applications (butyl rubber in the present embodiment), and is attached to the distal end of the cylindrical tip 3 by a tapered concave portion 8a that is formed concaved to the rear end side tightly fitting to the tapered outer peripheral surface 3j of the lure tip 3a.

Also, an insertion portion 8b that is inserted in the communication hole 3f when the rubber cap 8 is attached to the cylindrical tip 3 is formed extending in a rod shape from the bottom portion of the tapered concave portion 8a in the rubber cap 8 toward the opening of the tapered concave portion 8a, and the distal end of the insertion portion 8b is made to serve as a sealing portion 8c that contracts in diameter into a tapered shape.

Then, when attached to the cylindrical tip 3, the sealing portion 8c fits into the distal end opening of the through hole 7e in the protruding portion 7b of the rubber packing 7, in the communication hole 3f of the lure tip 3a, and blocks the through hole 7e.

In the combined container-syringe 1 constituted as described above, when unused it is put in a state of the liquid medicament m being filled in the cylinder 2, and the rubber cap 8 being attached to the distal end of the cylindrical tip 3. In this state, the liquid medicament m comes into direct contact only with the cylinder 2, the stopper 5, the rubber packing 7, and the sealing portion 8c.

Here, while the cylinder 2 is made of glass, the stopper 2, the rubber packing 7, and the sealing portion 8c are made of rubber for medical applications and pharmaceutical applications, that is, butyl rubber. All of these materials are chemically stable and non-corrosive with respect to the liquid medicament m, and so even if in contact with the liquid medicament m over a long period, they do not affect the liquid medicament, m by elution or the like.

Accordingly, by enclosing the liquid medicament m with only these corrosion-resistant members, it is possible to store the combined container-syringe 1 for a long time without degrading the quality of the liquid medicament m.

Also, when unused the liquid medicament m is enclosed by the rubber packing 7 and the sealing portion 8c at the distal end side of a cylinder 2, and moreover upon being used it is possible to introduce the liquid medicament in to the distal end side just by removing the rubber cap 8. For that reason, it is possible to perform the holding and introduction of the liquid medicament m with a simple constitution without carrying out special processing on the cylinder 2 and the cylindrical tip 3 that are conventionally used.

Accordingly, since there is no need to provide special constituent elements such as a bypass chamber or a front stopper, visual recognition in the cylindrical tip 3 is not hindered.

Therefore, in the case of performing nurse aspiration or a mixed injection, visual recognition of the existence of backflow of blood and liquid medicament and the color state thereof becomes simple, and so it becomes possible to perform injection preparation work more smoothly.

Moreover, there is no space that allows leftover liquid medicament after injection since there is no bypass chamber and front stopper as described above, and when the plunger rod 6 is pushed in and the stopper 5 has been moved until the cylinder 2 distal end, the stopper 5 abuts the abutment surface 7b of the rubber packing 7, and makes close contact while undergoing mutual deformation. For that reason, it becomes possible to completely introduce the liquid medicament m to the distal end side without allowing any to remain in the cylinder 2.

Above, the first embodiment of the combined container-syringe 1 according to the present invention was described in detail, but the present invention is not limited thereto and, provided it does not depart from that technical concept, includes various design modifications.

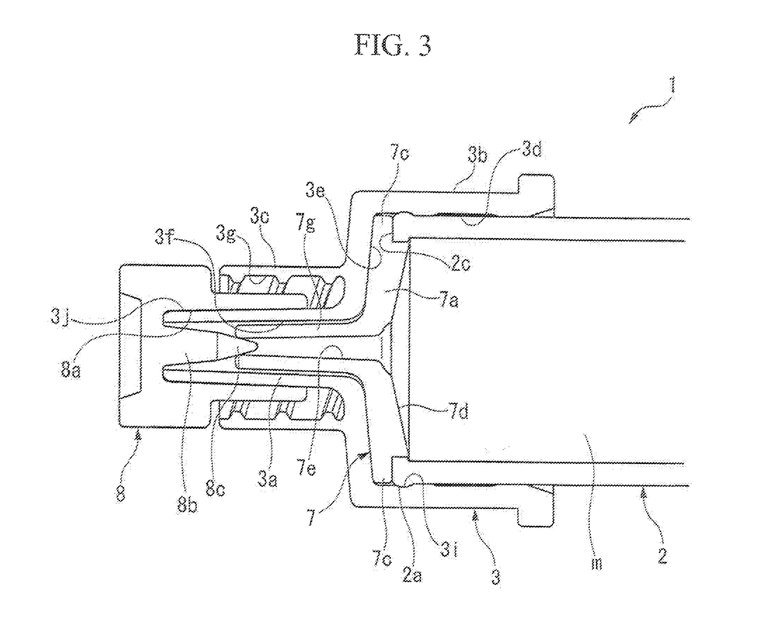

For example, as a first modification, it may be the combined container-syringe 1 as shown in FIG. 3. In this first modification, the length of the protruding portion 7b in the rubber packing 7 is formed longer than that of the combined container-syringe 1 of the aforementioned embodiment.

That is, while the length of the protruding portion 7b in the rubber packing 7 of the aforementioned embodiment is a length of one-fourth of the communication hole 3f, in the modification, a long protruding portion 7g is provided that has a length of about two-thirds of the communication hole 3f.

During an injection, the liquid medicament m is introduced to the needle while making contact with the through hole 7e of the rubber packing 7 and the communication hole 3f of the lure tip 3a, but if the protruding portion 7b is formed long as in this modification, it is possible to shorten the time that the liquid medicament m is in direct contact with the communication hole 3 f of the lure tip 3 a. For that reason, it becomes possible to introduce the liquid medicament in to the needle with the quality completely maintained,

Note that the protruding portion 7b and the long protruding portion 7d are not limited to the abovementioned lengths, and can be suitably changed.

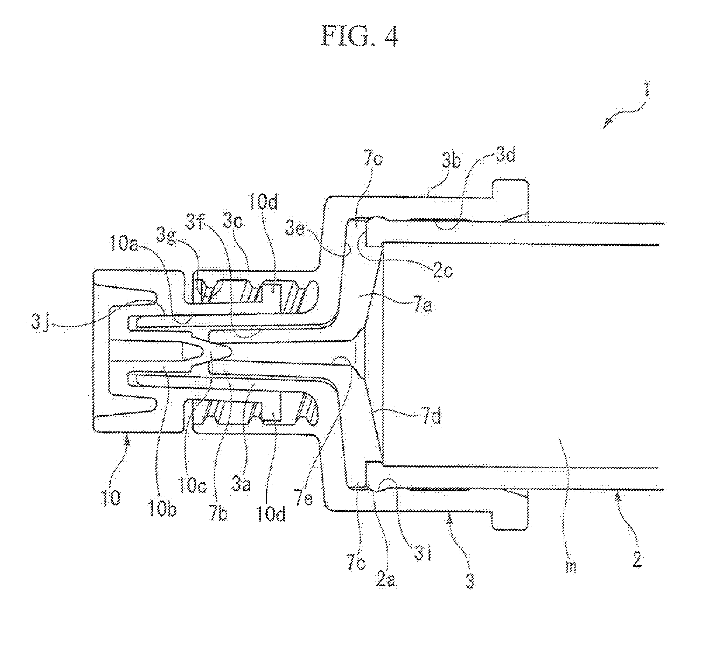

Also, as a second modification, for example a synthetic resin cap 10 may be provided as shown in FIG. 4 in place of the rubber cap 8 of the aforementioned embodiment. This synthetic resin cap 10 is formed with a synthetic resin that is stable with respect to a liquid medicament such as polypropylene, annular polyolefin, and annular polyolefin copolymer, and similarly to the rubber cap 8, is provided with a tapered concave portion 10a, an insertion portion 10b and a sealing portion 10c.

Also, unlike the rubber cap 8, the formation of this synthetic resin cap 10 is easy along with being provided with a certain degree of rigidity. For that reason, at the opening outer peripheral portion of the tapered concave portion 10a, a screw projection 10d is formed that protrudes toward the outside in the radial direction and is capable of screwing together with the lock screw 3g of the lure lock portion 3c.

In this synthetic resin cap 10, the tapered concave portion 10a is closely attached with the tapered outer peripheral surface 3j of the lure tip 3a, and by the screw projection 10d screwing together with the lock screw 3g, it can be firmly attached to the distal end of the cylindrical tip 3. Accordingly, it is possible to enhance the strength and shock resistance and it becomes possible to prevent a leakage of the liquid medicament m as a result the synthetic resin cap 10 accidentally slipping out.

Also, when the synthetic resin cap 10 is attached to the cylindrical tip 3, the sealing portion 10c blocks the through hole 7e, and the sealing portion 10c makes direct contact with the liquid medicament m. Here, polypropylene, annular polyolefin resin, and annular polyolefin copolymer all exhibit good corrosion resistance to the liquid medicament m. Accordingly, the sealing portion 10c of the synthetic resin cap 10 does not have an adverse influence on the liquid medicament m. Therefore, the combined container-syringe 1 according to the second modification can also be stored over a long period. Also, by moreover putting a cover (not illustrated) made of rubber for medical applications and pharmaceutical applications, that is, butyl rubber, on the sealing portion 10c of the synthetic resin cap 10, the material that makes contact with the liquid medicament m becomes only glass and rubber, and so a further increase in storability of the liquid medicament m may be achieved.

Note that in FIG. 4, although the rubber packing 7 is provided with the abovementioned long protruding portion 7d, it is not limited thereto and may also be one that is provided with an ordinary protruding portion 7 similarly to the embodiment shown in FIG. 1.

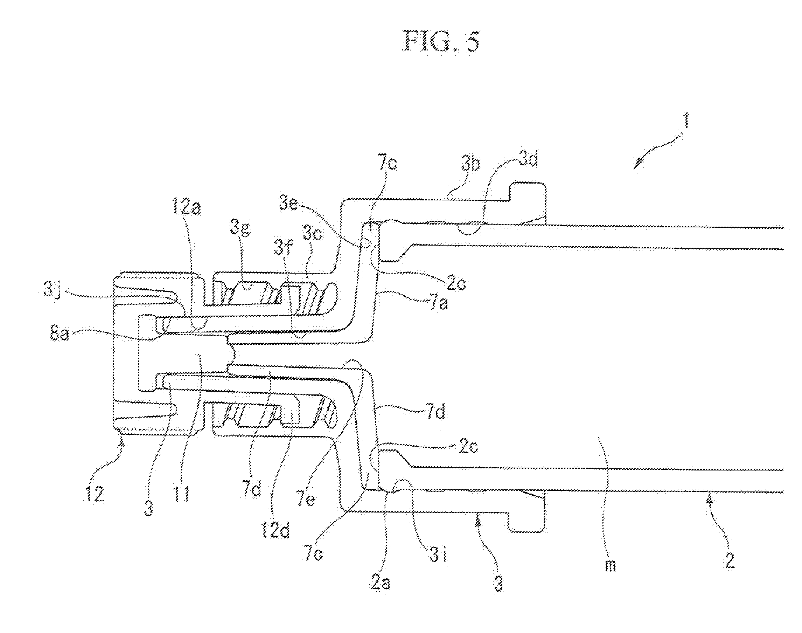

Also, as the third modification, one may be provided with a sealing rubber 11 and a fixing cap 12 that fixes the sealing rubber 11 as shown for example in FIG. 5 instead of the rubber cap 8 in the aforementioned embodiment.

The sealing rubber 11 consists of rubber for medical applications and pharmaceutical applications (butyl rubber), and is inserted into the communication hole 3f of the lure tip 3a for blocking the through hole 7e of the rubber packing 7. Then, she fixing cap 12 that consists of for example a material that has rigidity such as a synthetic resin or the like and is provided with a tapered concave portion 12a and a screw projection 12d is attached to the distal end of the cylindrical tip 3.

In this third modification, the sealing rubber 11 that the liquid medicament m makes contact with at the distal end of the through hole 7e of the rubber packing 7 is rubber for medical applications and pharmaceutical applications (butyl rubber), and similarly to the aforementioned embodiment, it is possible to seal the liquid medicament m with only materials that are chemically stable and non-corrosive with respect to the liquid medicament in, Moreover, the fixing cap 12 is firmly attached to the cylindrical tip 3 distal end by the close attachment of the tapered outer peripheral surface 3j of the lure tip 3a and tapered concave portion 12a, and being screwed together by the screw projection 12d.

Accordingly, similarly to the aforementioned embodiment, it is possible to store for a long time without degrading the liquid medicament m, and possible to prevent leakage of the liquid medicament m by enhancing the strength and shock resistance.

Also, as a fourth modification, for example as shown in FIG, 6, it may be one in which the protruding portion 7 is not formed in the rubber packing 7.

In this kind of rubber packing 7, in the case of using the synthetic resin cap 10, the insertion portion 1 0 extends to the vicinity of the opening of the communication hole 3f of the lure tip 3a, and the sealing portion 10c blocks the through hole 3e of the rubber packing 7.

This fourth modification is characterized by, in addition to being storable without deteriorating the liquid medicament, the formation being easy.

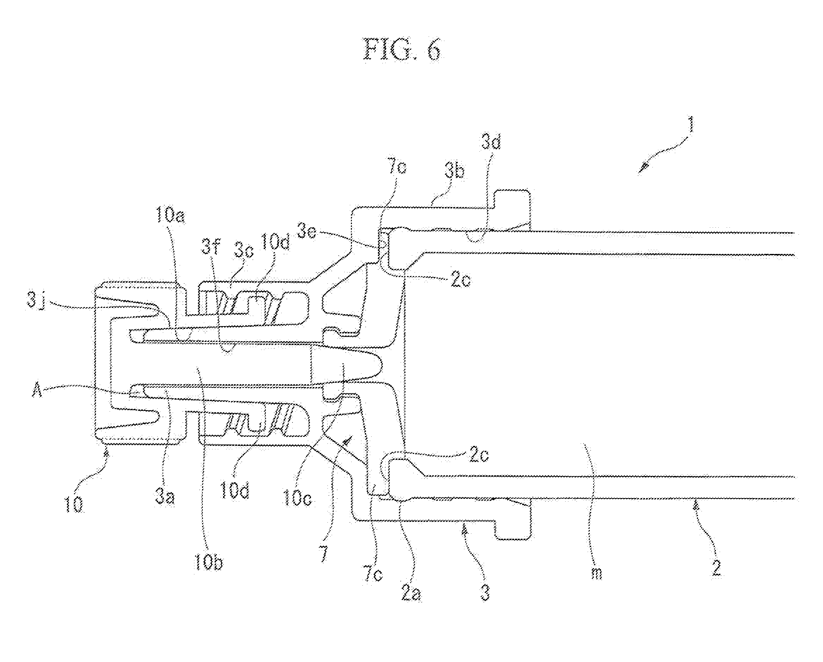

Also, in FIG. 6 the synthetic resin cap 10 is used, but the rubber cap 8 as in the embodiment shown in FIG. 1 may be used, and one that uses the sealing rubber 11 and the fixing cap 12 in combination as in the third modification may be used. Also, by covering the sealing portion 10c of the synthetic resin cap 10 with a cover (not illustrated) that consists of rubber for medical applications and pharmaceutical applications, that is, butyl rubber, the materials that come into contact with the liquid medicament m are only glass and rubber, and so a further increase in storability of the liquid medicament m may be achieved.

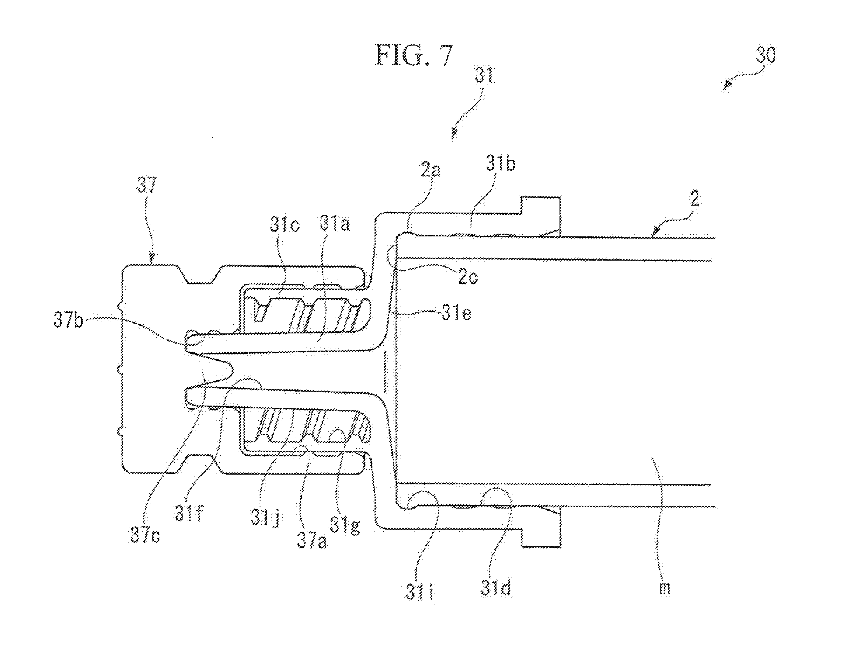

Next a combined container-syringe 30 according to the second embodiment of the present invention shall be described using FIG. 7. The combined container-syringe 30 of the present embodiment differs with respect to the rubber packing 7 that is interposed between the cylinder 2 and the cylindrical tip 3 in the combined container-syringe 1 of the first embodiment on the point of the distal end of the cylinder 2 being sealed by an integrated cylindrical tip 31 that is fitted from the outer side on the peripheral portion thereof, and on the other points has the same constitution as the first embodiment.

Note that in FIG. 7, the same reference numerals are given to the constituent elements that are the same as FIG. 2, and detailed descriptions thereof are omitted.

The integrated cylindrical tip 31 has an outer diameter two-stage columnar shape that consists of a synthetic resin that is chemically stable and non-corrosive with respect to the liquid medicament m such as polypropylene, annular polyolefin resin, and annular polyolefin copolymer, and is provided with a fitting portion 31b having a cylindrical shape, a lure tip 31a with a comparatively small diameter that is joined to the front side of the fitting portion 31b, and a lure lock portion 31c that is spaced to the outer side of the lure tip 31a in the radial direction and joined to the front side of the fitting portion 31b.

A bottomed fitting hole 31d is formed on the inner side of the fitting portion 31b, and by fitting the fitting hole 31d on the outer periphery of the distal end of the cylinder 2 from the outer side, the cylindrical tip 31 is attached in an air tight and liquid tight manner on the front end portion of the cylinder 2. Note that a ring-shaped groove 31i is formed on the front end side of the fitting hole 31d, and by fitting this together with the ring-shaped projection 2b of the distal end portion of the cylinder 2, the integrated cylindrical tip 31 is attached in an air tight and liquid tight manner on the distal end of the cylinder 2.

Also, the outer peripheral surface of the lure tip 31a is made into a tapered outer peripheral surface 31j that contracts in diameter while heading to the distal end side, and a communication hole 31f that is continuous with a bottom portion 31e of the 31d is formed penetrating in the front/rear direction through the inside of the lure tip 31a along the axis line.

Furthermore, a lock screw 31g for engaging the needle base of the needle not illustrated is formed in the above-mentioned lure lock portion 31c.

A rubber cap 37 is attached to the distal end side of the integrated cylindrical tip 31. The rubber cap 37 consists of rubber for medical applications and pharmaceutical applications (butyl rubber in the present embodiment), and is provided with a first concave portion 37a that is formed so as to be indented from the rear end side thereof and which the outer peripheral surface of the lure lock portion 31c fits into, and a second concave portion 37b that is formed so as to be further indented from the center of the bottom portion of the first concave portion 37a and which the tapered outer peripheral surface 31j of the lure tip 31a fits into. By fitting together the first concave portion 37a and the second concave portion 37b with the lure lock portion 31c and the lure tip 31a, the rubber cap 37 is attached to the distal end of the integrated cylindrical tip 31.

Also, a sealing portion 37c that is formed so as to protrude in a tapered shape toward the opening of the second concave portion 37b is formed at the bottom portion of the second concave portion 37b. The sealing portion 37c has the role of sealing the liquid medicament m by blocking in an air tight and liquid tight manner the communication hole 31f when the rubber cap 37 is attached to the integrated cylindrical tip 31.

In the combined container-syringe 30 that is provided with this integrated cylindrical tip 31, when unused it is put in a state of the liquid medicament m being filled in the cylinder 2, and the rubber cap 37 being attached to the distal end of the integrated cylindrical tip 31. In this state, the liquid medicament m comes into direct contact only with the cylinder 2, the stopper 5, the sealing portion 37c and the integrated cylindrical tip 31.

Accordingly, similarly to the first embodiment, since it is possible to seal the liquid medicament m with only members that are chemically stable and non-corrosive with respect to the liquid medicament m, it is possible to store the combined container-syringe 30 for a long period.

Also, since the integrated cylindrical tip 31 is adopted, it is possible to constitute the combined container-syringe 30 with fewer constituent elements compared to the first embodiment, and it is possible to achieve an increase in productivity and a reduction in production costs.

In addition, similarly to the first embodiment, besides being able to ensure visibility in the integrated cylindrical tip 31 and the cylinder 2, it is possible to prevent the liquid medicament m from remaining in the cylinder 2 after the completion of the injection.

Note that even in the second embodiment of the combined container-syringe of the present invention, similarly to the aforementioned first embodiment, needless to say the present invention is not to be limited thereto and includes some design modifications provided it does not depart from that technical concept of the present invention.

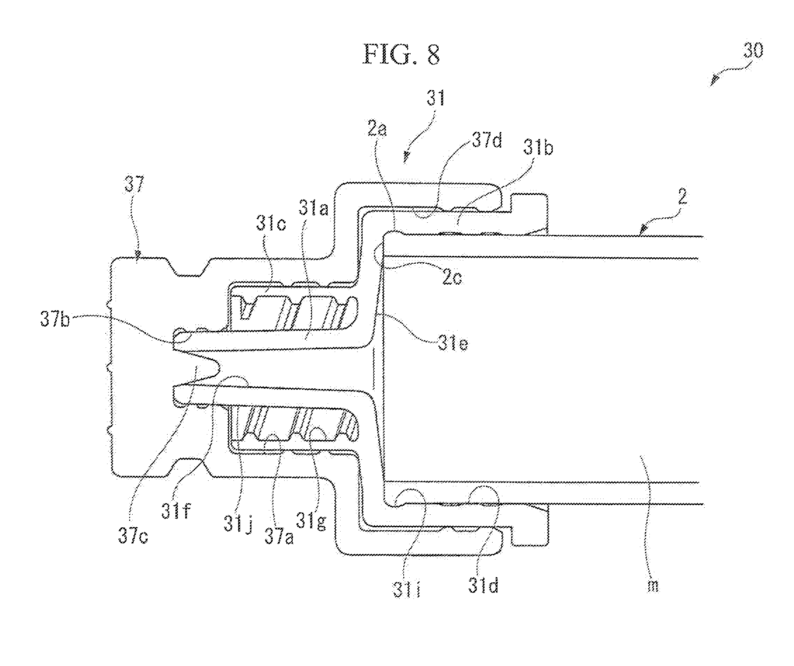

For example, as a modification of the second embodiment, as shown in FIG. 8, the rubber cap 37 may be one that covers the whole of the integrated cylindrical tip 31.

That is, in the rubber cap 37 in the present modification, by enlarging the diameter by one step more than the first concave portion 37a, a large diameter concave portion 37d that the periphery of the fitting portion 31d in the integrated cylindrical tip 31 fits into is formed.

Thereby, when the rubber cap 37 is attached, since the whole of the periphery of the integrated cylindrical tip 31 is covered, it is possible to reliably prevent leakage of the liquid medicament m and intrusion of outside area at the distal end of the lure tip 31a.

### INDUSTRIAL APPLICABILITY

According to the present invention, it is possible to provide a combined container-syringe that is as safe and easy to use as a conventional combined container-syringe and capable of being stored for a long time without degrading a liquid medicament, and also enables good observation of a liquid medicament and blood that flows into the cylindrical tip and moreover is capable of preventing leftover liquid medicament after injection.

## Claims

1. A combined container-syringe comprising: a cylinder made of glass that has a cylindrical shape; a stopper that is fitted into a rear end side of the cylinder in a liquid tight manner; a cylindrical tip that is formed with a transparent synthetic resin material, fitted from an outer side on a distal end outer periphery of the cylinder via a fitting hole on a base end side, and provided with a lure tip to which a needle is attached at the distal end side and in which a communication hole that communicates with the inside of the cylinder is formed; a finger grip that is provided at the rear end of the cylinder; and a plunger rod that is inserted in the cylinder from the rear end side of the cylinder and coupled to the stopper,
wherein a rubber packing that covers the distal end opening of the cylinder at the fitting hole in an air tight and liquid tight manner, and in which a through hole that brings the communication hole and the inside of the cylinder into communication is formed and
a sealing member that is inserted from the distal end side of the communication hole and blocks the through hole are provided, and
the stopper and the rubber packing are formed with elastic bodies that are corrosion resistant to the liquid medicament, and the liquid medicament is filled between the stopper and the rubber packing in the cylinder.

2. The combined container-syringe according to claim 1, wherein a peripheral edge of the through hole in the rubber packing extends to the inside of the communication hole, and covers the inner peripheral surface of the communication hole.

3. The combined container-syringe according to claim 1, wherein the stopper and the rubber packing are made of butyl rubber.

4. The combined container-syringe according to claim 1, wherein the sealing member is made of butyl rubber.

5. The combined container-syringe according to claim 1, wherein the sealing member is made of a synthetic resin that has corrosion resistance to the liquid medicament.

6. The combined container-syringe according to claim 5, wherein the synthetic resin is any one of polypropylene, annular polyolefin, and annular polyolefin copolymer.

7. The combined container-syringe according to claim 1, wherein the front end portion of the cylinder extends toward the inner side in the radial direction of the cylinder, whereby the distal end opening portion of the cylinder contracts in diameter, and
the rubber packing, by making contact with the entire region of the front end portion of the cylinder, covers the distal end opening of the cylinder in an air tight and liquid tight manner.

8. A combined container-syringe comprising: a cylinder made of glass that has a cylindrical shape; a stopper that is fitted into a rear end side of the cylinder in a liquid tight manner; a cylindrical tip that is formed with a synthetic resin material, fitted from an outer side on a distal end outer periphery of the cylinder via a fitting hole on a base end side, and provided with a lure tip to which a needle is attached at the distal end side and in which a communication hole that communicates with the inside of the cylinder is formed; a finger grip that is provided at the rear end of the cylinder; and a plunger rod that is inserted in the cylinder from the rear end side of the cylinder and coupled to the stopper,
wherein a sealing member that blocks the communication hole is provided, and
the sealing member and the stopper are formed with elastic bodies having corrosion resistance to the liquid medicament, at least the contact location with the liquid medicament at the cylindrical tip is formed with a synthetic resin that has corrosion resistance to the liquid medicament, and the liquid medicament is filled between the stopper in the cylinder and the sealing member and the cylindrical tip.

9. The combined container-syringe according to claim 8, wherein the synthetic resin that has corrosion resistance to the liquid medicament is any one of polypropylene, annular polyolefin, and annular polyolefin copolymer.

10. The combined container-syringe according to claim 8, wherein the sealing member covers the entire region of the outer peripheral surface of the cylindrical tip.

11. The combined container-syringe according to claim 8, therein the sealing member and the stopper are made of butyl rubber.
